# EUROPÄISCHE PATENTSCHRIFT

(11) **EP 1 125 923 B1**
(45) Veröffentlichungstag und Bekanntmachung des Hinweises auf die Patenterteilung: **25.05.2005**
(21) Anmeldenummer: 01101420.6
(22) Anmeldetag: 23.01.2001
(51) Int. Cl.: C07D 207/267

(54) **Verfahren zur Herstellung von N-Methyl-2-Pyrrolidon (NMP)**
Process for the preparation of N-methyl-2-pyrrolidinone (NMP)
Procédé pour la préparation de N-méthyl-2-pyrrolidinone (NMP)

(30) Priorität: 04.02.2000 DE 10004909
(43) Veröffentlichungstag der Anmeldung: 22.08.2001
(73) Patentinhaber: BASF AKTIENGESELLSCHAFT, 67056 Ludwigshafen (DE)
(72) Erfinder: Ohlbach, Frank, Dr., 69221 Dossenheim (DE); Melder, Johann-Peter, Dr., 67459 Böhl-Iggelheim (DE); Ross, Karl-Heinz, Dr., 67269 Grünstadt (DE); Rudloff, Martin, 67273 Weisenheim (DE); Liebe, Jörg, Dr., 158-0086 Tokyo (JP)

(56) Entgegenhaltungen:
- DATABASE WPI Section Ch, Week 198936 Derwent Publications Ltd., London, GB; Class E13, AN 1989-260914 XP002201297 & JP 01 190667 A (MITSUBISHI KASEI CORP), 31. Juli 1989 (1989-07-31) -& CHEMICAL ABSTRACTS, vol. 112, no. 11, 12. März 1990 (1990-03-12) Columbus, Ohio, US; abstract no. 98377g, Seite 735; XP002201295
- B. ELVERS ET AL. (ED.): "Ullmann's Encyclopedia of Industrial Chemistry, 5th ed., vol. A 16" 1990 , VCH VERLAGSGESELLSCHAFT MBH , WEINHEIM, DE; XP002201292 * Seite 535 - Seite 541 *
- DATABASE WPI Section Ch, Week 198936 Derwent Publications Ltd., London, GB; Class E13, AN 1989-259000 XP002201298 & JP 01 186864 A (MITSUBISHI KASEI CORP), 26. Juli 1989 (1989-07-26)
- DATABASE WPI Section Ch, Week 198936 Derwent Publications Ltd., London, GB; Class E13, AN 1989-258999 XP002201299 & JP 01 186863 A (MITSUBISHI KASEI CORP), 26. Juli 1989 (1989-07-26)
- DATABASE WPI Section Ch, Week 199834 Derwent Publications Ltd., London, GB; Class E13, AN 1998-393443 XP002201300 & JP 10 158238 A (TONEN KAGAKU KK), 16. Juni 1998 (1998-06-16)
- DATABASE WPI Section Ch, Week 199112 Derwent Publications Ltd., London, GB; Class A60, AN 1991-085402 XP002201301 & SU 1 558 903 A (LENGD LENNEFTEKHIM), 23. April 1990 (1990-04-23)
- B. ELVERS ET AL. (ED.): "Ullmann's Encyclopedia of Industrial Chemistry, 5th ed., vol. A 22" 1993 , VCH VERLAGSGESELLSCHAFT MBH , WEINHEIM, DE; XP002201293 * Seite 458 - Seite 459 *
- I. KROSCHWITZ ET AL. (ED.): "Kirk-Othmer: Encyclopedia of Chemical Technology, 4th ed., vol. 2" 1992 , JOHN WILEY & SONS , NEW YORK XP002201294 * Seite 373 - Seite 375 *
- CHEMICAL ABSTRACTS, vol. 134, no. 13, 26. März 2001 (2001-03-26) Columbus, Ohio, US; abstract no. 178463b, Seite 705; XP002201296 -& RO 113 640 B (CHIMCOMPLEX S.A.) 30. September 1998 (1998-09-30)

## Beschreibung

Die vorliegende Erfindung betrifft ein Verfahren zur Herstellung von N-Methyl-2-pyrrolidon (= 1-Methyl-2-pyrrolidinon, NMP)

NMP eignet sich wegen seiner leichten Flüchtigkeit, thermischen Stabilität, hohen Polarität und aprotischen Eigenschaften als Lösungsmittel für Polymere sowie als Lösungsmittel für zahlreiche organische Synthesen, wie z. B. Alkylierungen oder Herstellung von Carbonsäuren und ihren Derivaten.

Technische Bedeutung hat NMP vor allem für die Trennung von Acetylen aus Spaltgas oder von Butadien aus C₄-Schnitten, für die Extraktion von Aromaten oder zur Absorption saurer Bestandteile bei Gaswäschen.

Die technische Herstellung von NMP erfolgt überwiegend durch Umsetzung von gamma-Butyrolacton (γ-BL) mit Monomethylamin (MMA) in einem Rohrreaktor, z. B. Schaftreaktor, bei 200 bis 350 °C und erhöhtem Druck, z. B. ca. 10 MPa, (Ullmann's Encyclopedia of Industrial Chemistry, 5. Ed., Vol. A22, Seiten 458 bis 459 (1993)).

Beispielsweise beschreibt JP-A-10 158 238 (Derwent Abstr. 98-393443/34) die Umsetzung von γ-BL mit überschüssigem MMA bei 250 bis 300 °C in Gegenwart von Wasser zu NMP.

JP-A-1 190 667 (Derwent Abstr. 89-260914/36) beschreibt die Herstellung von NMP durch Reaktion von γ-BL mit überschüssigem MMA, wobei das nach der Reaktion unumgesetzt gebliebene MMA sowie als Nebenprodukt erhaltenes Dimethylamin (DMA) und Trimethylamin (TMA) zusammen mit hinzugefügtem Wasser wieder in die Reaktion von γ-BL mit überschüssigem MMA zurückgeführt werden.

JP-A-7 218 751 (Derwent Abstr. 35795T-E) berichtet über die Synthese von NMP durch erhitzen von γ-BL, oder offenkettigen Derivaten hiervon, mit DMA und/oder TMA bei Temperaturen größer 200 °C. In einem Beispiel liefert die Umsetzung von γ-BL mit wässrigem DMA bei 270 °C / 3 h NMP mit einer Ausbeute von 80 %.

JP-A-1 186 864 (Derwent Abstr. 89-259000/36) offenbart die Herstellung von N-alkylierten Lactamen durch Umsetzung entsprechender Lactone mit sekundären Aminen in Gegenwart von Wasser über die entsprechenden N,N-Dialkyl-omega-hydroxycarboxamide als Zwischenstufe. Gemäß Beispiel 1 dieser Patentanmeldung liegt bei der Umsetzung von γ-BL mit wässrigem DMA die NMP-Ausbeute bei 60 % und zusätzlich werden γ-Hydroxybuttersäuremethylamide gebildet. Auch in dem einzigen weiteren anmeldungsgemäßen Beispiel wird bei der entsprechenden Umsetzung von γ-BL mit DMA über eine NMP-Ausbeute von 60 % berichtet.

JP-A-1 186 863 (Derwent Abstr. 89-258999/36) beschreibt die Herstellung von N-alkylierten Lactamen durch Reaktion von entsprechenden Lactonen mit tertiären Aminen oder mit tertiären oder quartären Ammoniumverbindungen in Gegenwart von Wasser und unter Eliminierung eines entsprechenden Alkohols. Gemäß Beispiel 1 dieser Patentanmeldung liegt bei der Umsetzung von γ-BL mit wässrigem TMA die NMP-Ausbeute bei 8 % und große Mengen an Nebenprodukten wie γ-Hydroxybuttersäuremethylamide, 2-Pyrrolidon und γ-Hydroxybuttersäure werden gebildet.

Die Methylamine Monomethylamin (MMA), Dimethylamin (DMA) und Trimethylamin (TMA) werden technisch in einem kontinuierlichen Verfahren durch die (exotherme) Reaktion von Ammoniak mit Methanol in Gegenwart eines Katalysators bei erhöhter Temperatur hergestellt (z. B.: Kirk-Othmer, Encyclopedia of Chemical Technology, 4. Ed., Vol. 2, Seiten 373 bis 375 (1992) und Ullmann's Encyclopedia of Industrial Chemistry, 5. Ed., Vol. A16, Seiten 535 bis 541 (1990)).

Als Katalysatoren werden saure Katalysatoren, insbesondere feste saure Katalysatoren, wie Siliciumoxide (Silica, SiO₂), Aluminiumoxide (Alumina, Al₂O₃), Silica-Alumina (SiO₂ • Al₂O₃), Titanoxide (Titania, TiO₂), Wolframoxide, Phosphate (AlPO₄), Zeolithe und Tone, (Methode 1) oder Metallkatalysatoren, wie kobalt-, nickeloder kupferhaltige Katalysatoren (z. B. Kupferchromit), (Methode 2) eingesetzt. Der Katalysator ist üblicherweise als Festbett angeordnet.

Die Reaktionstemperaturen liegen in der Regel bei Methode 1 bei 300 bis 500 °C, insbesondere bei 390 bis 430 °C, und bei Methode 2 bei 130 bis 250 °C.

Bei Methode 1 liegt der Druck in der Regel bei 790 bis 3550 kPa, insbesondere bei 1500 bis 3000 kPa; Methode 2 wird meist in Gegenwart von Wasserstoff durchgeführt.

Bei diesen Umsetzungen von Ammoniak mit Methanol wird stets ein Gemisch der Methylamine MMA, DMA und TMA zusammen mit Wasser erhalten. Die Gesamtselektivität zu den Methylaminen beträgt etwa 94 %; Nebenreaktionen sind Spaltungen zu CO, CO₂, CH₄, H₂ und N₂. (vergl. z. B. K. Weissermel et al., Industrielle Organische Chemie, 3. Auflage, Seiten 53 bis 54 (1990)).

Das Reaktionsrohprodukt, enthaltend im Wesentlichen Ammoniak, Wasser, gegebenenfalls unumgesetztes Methanol und die Methylamine, wird durch eine kontinuierliche mehrstufige, technisch aufwendige, Destillation aufgetrennt (Kombination verschiedener Druck- und Extraktivdestillationen). Ein typisches Verfahrensschema für die Synthese der Methylamine und deren Isolierung ist die Fig. 2. in Kirk-Othmer, Encyclopedia of Chemical Technology, 4. Ed., Vol. 2, Seite 375 (1992), auf die hiermit Bezug genommen wird.

Aus dem Reaktionsgemisch wird beispielsweise in einer insgesamt technisch aufwendigen Destillationssequenz zunächst Ammoniak und ein Teil des TMAs und anschließend in einer Extraktionskolonne mit Wasser das restliche TMA abgetrennt. Ammoniak wird in die Reaktion rückgeführt. In einer anschließenden Entwässerungskolonne wird MMA und DMA über Kopf abgetrennt und in einer separaten Kolonne aufgetrennt. Methanol wird aus einem Seitenstrom der Entwässerungskolonne entnommen und in einer separaten Kolonne von Wasser getrennt und in die Synthese rückgeführt. DMA und TMA können ebenfalls grundsätzlich in die Reaktion von NH₃ mit Methanol rückgeführt werden, wobei aus DMA und TMA unter den Reaktionsbedingungen jeweils wieder Gemische aus MMA, DMA und TMA entstehen (thermodynamisches Gleichgewicht; vergl. z. B. Ullmann's Encyclopedia of Industrial Chemistry, 5. Ed., Vol. A16, Seite 537 (1990)).

Weitere Verfahrensausgestaltungen des Methylamin-Herstellprozesses finden sich im Dokument J. Ramioulle et al., Hydrocarbon Processing, July 1981, Seiten 113 bis 117 (siehe z. B. dort Fig. 6 auf Seite 117), auf das hiermit ebenfalls Bezug genommen wird.

Nachteilig an den Verfahren des Stands der Technik zur Herstellung von NMP ausgehend von Ammoniak, Methanol und γ-BL ist, dass man das für die Umsetzung mit γ-BL benötigte MMA zuerst aus dem Reaktionsrohaustrag der Synthese der Methylamine durch eine technisch sehr aufwendige Destillationskaskade (Hintereinanderschaltung mehrerer Destillationskolonnen) (wie oben beschrieben) isolieren muss.

Der vorliegenden Erfindung lag daher unter Überwindung der Nachteile des Stands der Technik die Aufgabe zugrunde, ein effizientes, selektives, wirtschaftliches und technisch weniger aufwendiges Verfahren zur Herstellung von NMP, ausgehend von Ammoniak, Methanol und γ-BL, in hohen Ausbeuten (bezogen auf γ-BL) und Raum-Zeit-Ausbeuten aufzufinden.

Demgemäß wurde ein Verfahren zur Herstellung von N-Methyl-2-pyrrolidon (NMP) gefunden, welches dadurch gekennzeichnet ist, dass man in einer ersten Verfahrensstufe durch Reaktion von Ammoniak mit Methanol bei erhöhter Temperatur in Gegenwart eines Katalysators ein Gemisch enthaltend Mono-, Di- und Trimethylamin und Ammoniak herstellt, den Ammoniak abtrennt, das Gemisch enthaltend die Methylamine in einer zweiten Verfahrensstufe bei erhöhter Temperatur und erhöhtem Druck mit gamma-Butyrolacton (γ-BL), wobei das molare Verhältnis von Monomethylamin zu γ-BL mindestens 1 ist, umsetzt, NMP und unumgesetzte Methylamine aus dem Umsetzungsprodukt abtrennt und unumgesetzte Methylamine in die erste Verfahrensstufe zur Reaktion mit Methanol und Ammoniak zurückführt.

Erfindungsgemäß kann auf die technisch sehr aufwendige Auftrennung des Reaktionsrohprodukts der Umsetzung von Methanol mit Ammoniak in die einzelnen Methylamine MMA, DMA und TMA oder in die entsprechenden binären Gemische (z. B. MMA + DMA) verzichtet werden.

### Das erfindungsgemäße Verfahren lässt sich wie folgt ausführen:

Durch die Umsetzung von Ammoniak mit Methanol in Gegenwart eines sauren Katalysators, besonders bevorzugt festen sauren Katalysators (wie z. B. AlOₓ), oder eines Metallkatalysators, wobei der Katalysator besonders bevorzugt im Reaktor als Festbett angeordnet ist, bei erhöhter Temperatur wird gemäß den bekannten Verfahren des Stands der Technik, die oben beschrieben wurden, ein Gemisch enthaltend im Wesentlichen die drei Methylamine MMA, DMA und TMA, Ammoniak, Wasser und gegebenenfalls unumgesetztes Methanol hergestellt. (Erste Verfahrensstufe).

"Im Wesentlichen" bedeutet in diesem Zusammenhang, dass der Gehalt an den drei Methylaminen, Ammoniak, Wasser und gegebenenfalls Methanol im Gemisch insgesamt mindestens 95 Gew.-%, bevorzugt mindestens 97 Gew.-%, insbesondere mindestens 98 Gew.-%, besonders bevorzugt mindestens 99 Gew.-%, beträgt.

Aus dem Gemisch enthaltend im Wesentlichen die drei Methylamine, Ammoniak, Wasser und gegebenenfalls unumgesetztes Methanol wird anschließend der Ammoniak gemäß den bekannten Verfahren in einer Destillationskolonne über Kopf abgetrennt (siehe z. B. Fig. 2. in Kirk-Othmer, Encyclopedia of Chemical Technology, 4. Ed., Vol. 2, Seite 375: erste Kolonne nach dem Reaktor).

Dabei wird ein Gemisch enthaltend im Wesentlichen die drei Methylamine, Wasser und gegebenenfalls Methanol erhalten. "Im Wesentlichen" bedeutet in diesem Zusammenhang, dass der Gehalt an den drei Methylaminen, Wasser und gegebenenfalls Methanol im Gemisch insgesamt mindestens 96 Gew.-%, bevorzugt mindestens 97 Gew.-%, insbesondere mindestens 98 Gew.-%, besonders bevorzugt mindestens 99 Gew.-%, beträgt.

Der Gehalt an Wasser in diesem Gemisch beträgt im allgemeinen 30 bis 50 Gew.-%, bevorzugt 35 bis 45 Gew.-%.

Der Gehalt an Methanol in diesem Gemisch beträgt im allgemeinen 0 bis 10 Gew.-%, bevorzugt 3 bis 7 Gew.-%.

Der Restgehalt an Ammoniak in diesem Gemisch beträgt im allgemeinen 0 bis 1 Gew.-%, bevorzugt 0,1 bis 0,8 Gew.-%.

Das Gewichtsverhältnis der Methylamine in diesem Gemisch beträgt im allgemeinen MMA : DMA : TMA = (1 bis 14) : (6 bis 12) : (0,2 bis 12), bevorzugt MMA : DMA : TMA = (1 bis 2,7) : (2 bis 3) : (0,07 bis 2).

In einer sich anschließenden Extraktionskolonne und einer oder mehrerer Destillationskolonnen kann gewünschtenfalls gemäß den bekannten Verfahren aus diesem Gemisch gegebenenfalls vorhandenes Methanol über Kopf und Wasser über Sumpf abgereichert oder abgetrennt werden (siehe z. B. Fig. 2. in Kirk-Othmer, Encyclopedia of Chemical Technology, 4. Ed., Vol. 2, Seite 375: zweite und dritte Kolonne nach dem Reaktor).

Das nach den obigen Schritten erhaltene Gemisch enthaltend im Wesentlichen die drei Methylamine MMA, DMA und TMA, gegebenenfalls Wasser und gegebenenfalls Methanol wird bei erhöhter Temperatur, bevorzugt bei Temperaturen von 180 bis 350 °C, insbesondere bei Temperaturen von 200 bis 300 °C, besonders bevorzugt bei Temperaturen von 230 bis 270 °C, und bei erhöhtem Druck, bevorzugt bei Drucken von 5 bis 300 bar, insbesondere von 50 bis 150 bar, mit gamma-Butyrolacton (γ-BL) umgesetzt. (Zweite Verfahrensstufe)

Das molare Verhältnis von MMA zu γ-BL beträgt hierbei mindestens 1, bevorzugt mindestens 1,05, besonders bevorzugt mindestens 1,1. Bevorzugte Bereiche für das molare Verhältnis von MMA zu γ-BL sind 1 bis 2, bevorzugt 1,05 bis 1,5, besonders bevorzugt 1,1 bis 1,25.

Die Umsetzung kann diskontinuierlich in einem Druckreaktor (Autoklav) oder bevorzugt kontinuierlich in einem Rohrreaktor, der Einbauten zur Beeinflussung der Strömungsverhältnisse im Reaktor aufweisen kann, z. B. in einem Schaftreaktor, erfolgen.

Die Verweilzeit des Reaktionsgemisches im Reaktor unter den angegebenen Bedingungen beträgt im allgemeinen 1 bis 6 Stunden, bevorzugt 1,5 bis 5 Stunden, besonders bevorzugt 2 bis 4 Stunden.

Für diese Umsetzung kommt bevorzugt ein gamma-Butyrolacton (γ-BL) mit einer Reinheit von mindestens 98 Gew.-%, bevorzugt mindestens 99 Gew.-% zum Einsatz.

Das benötigte γ-BL kann nach bekannten Verfahren durch die endotherme cyclisierende Dehydrierung von 1,4-Butandiol in der Gasphase an einem Metallkatalysator (z. B. Kupferkatalysator) bei erhöhter Temperatur oder durch selektive Hydrierung von Maleinsäureanhydrid bei erhöhtem Druck und erhöhter Temperatur an einem Metallkatalysator und jeweils anschließender destillativer Reinigung erhalten werden (Ullmann's Encyclopedia of Industrial Chemistry, 5. Ed., Vol. A4, Seite 496 (1985)).

Das erhaltene Reaktionsprodukt besteht aus NMP, unumgesetzten Methylaminen (insbesondere unumgesetztem DMA und TMA), Wasser, geringen Mengen an Nebenprodukten, gegebenenfalls Methanol und gegebenenfalls unumgesetztem γ-BL.

In der Regel beträgt der γ-BL-Umsatz in dieser Verfahrensstufe unter den angegebenen Reaktionsbedingungen größer 95 %, insbesondere größer 97 %, ganz besonders größer 99 %.

Typischerweise beträgt der Gehalt an Nebenprodukten im Reaktionsprodukt kleiner 5 Gew.-%, insbesondere kleiner 3,5 Gew.-%, ganz besonders kleiner 2 Gew.-%.

Das gewünschte Verfahrensprodukt NMP wird aus dem Reaktionsprodukt durch eine fraktionierte ein- oder mehrstufige Destillation isoliert.

Beispielsweise kann die destillative Aufarbeitung des Reaktionsprodukts zweistufig ausgeführt werden, wobei in der ersten Destillationsstufe als Kopfdestillat DMA und TMA und gegebenenfalls noch vorhandenes MMA abgezogen werden und in der zweiten Destillationsstufe Wasser über Kopf und das reine NMP über einen Seitenabzug abgetrennt werden.

Die bei der destillativen Aufarbeitung des Reaktionsprodukts anfallenden Methylamine, insbesondere DMA und TMA, werden erfindungsgemäß in die erste Verfahrensstufe der Reaktion von Ammoniak mit Methanol zurückgeführt.

Auch das anfallende Methanol kann entsprechend in die erste Verfahrensstufe zurückgeführt werden.

Im erfindungsgemäßen Verfahren beträgt bei γ-BL-Umsätzen größer 95 % die Selektivität für die Bildung von NMP (bezogen auf γ-BL) größer 90 %, insbesondere größer 93 %, ganz besonders mindestens 95 %.

Da im Stand der Technik bekannt war, dass auch DMA und TMA mit γ-BL reagieren, dabei jedoch jeweils nur in sehr schlechten Selektivitäten und Ausbeuten NMP erhalten wird, weil Nebenprodukte gebildet werden, ist es überraschend, dass mit dem erfindungsgemäßen Verfahren, in dem MMA, DMA und TMA mit γ-BL umgesetzt werden und anschließend unumgesetzte Methylamine in die Reaktion von NH₃ mit Methanol zurückgeführt werden, sehr hohe Selektivitäten und Ausbeuten für die Bildung von NMP (bezogen auf γ-BL) erzielt werden.

Dieser Sachverhalt wird durch die unten aufgeführten Beispiele 1 bis 4 verdeutlicht.

Die unten aufgeführten Beispiele 1 und 2 zeigen, dass sich γ-BL jeweils bereits bei 5 °C bis Raumtemperatur nahezu quantitativ mit wässrigen Lösungen der überschüssigen Methylamine MMA und/oder DMA zu den entsprechenden γ-Hydroxybuttersäuremethylamiden umsetzt.

Beispiel 2 zeigt, dass sich die jeweils aus γ-BL und MMA oder DMA erhaltenen Amide γ-Hydroxybuttersäuremonomethylamid oder γ-Hydroxybuttersäuredimethylamid und das aus γ-BL und TMA bereits bei Raumtemperatur erhaltene innere Salz ("TMA-BL-Addukt") der Formel 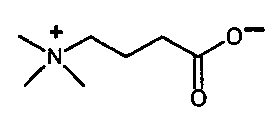 bei erhöhter Temperatur, vorzugsweise bei Temperaturen größer 200 °C, durch Cyclisierung und Abspaltung von Wasser bzw. Methanol in NMP umwandeln lassen.

Erfindungsgemäß wurde erkannt, dass bei vergleichbaren Bedingungen die Cyclisierung des Monomethylamids zu NMP deutlich schneller als die des Dimethylamids bzw. des TMA-BL-Addukts verläuft. Weiterhin wurde gefunden, dass die Cyclisierung zum NMP sowohl in wässriger Lösung als auch wasserfrei erfolgen kann. Unter wasserfreien Bedingungen sind die NMP-Ausbeuten ausgehend von DMA oder TMA deutlich niedriger als wenn die Umsetzungen in Gegenwart von Wasser durchgeführt werden (Beispiele 2a und 2b).

Die Ausbeuten an NMP durch Umsetzung von DMA oder TMA mit γ-BL bei 255 °C / 3 h liegen gemäß Beispiel 2 aber nur bei höchstens 63 %. Der γ-BL-Umsatz ist in diesen Umsetzungen mit DMA oder TMA in jedem Fall wesentlich höher als die entsprechende NMP-Ausbeute, da im Umsetzungsprodukt erhebliche Mengen an Nebenprodukten, wie z. B. γ-Hydroxybuttersäuredimethylamid bzw. TMA-BL-Addukt, vorhanden sind.

Erfindungsgemäß wurde weiterhin erkannt (siehe Beispiel 3), dass sich γ-Hydroxybuttersäuremonomethylamid und γ-Hydroxybuttersäuredimethylamid in Gegenwart von überschüssigem MMA bzw. DMA ab Temperaturen von etwa 80 °C sehr leicht ineinander umwandeln, sich also bei erhöhter Temperatur ein thermodynamisches Gleichgewicht zwischen den beiden Amiden einstellt.

Es wurde zudem erfindungsgemäß erkannt (Beispiel 4), dass sich im Fall der Umsetzung eines äquimolaren Gemisches von MMA und DMA mit einem molaren Unterschuss an γ-BL (bezogen auf MMA + DMA), wobei MMA mindestens in äqimolarer Menge bezogen auf γ-BL vorliegt, zunächst bei Raumtemperatur kinetisch kontrolliert ein Gemisch aus den beiden entsprechenden Amiden (Hydroxybuttersäuremonomethylamid und γ-Hydroxybuttersäuredimethylamid) im Verhältnis von etwa 3 : 1 zugunsten des Monomethylamids bildet. Durch Erhöhung der Temperatur auf über 80 °C stellt sich das thermodynamische Gleichgewicht zwischen den beiden entsprechenden Amiden ein, welches noch deutlicher auf der Seite des Monomethylamids liegt. Bei 270 °C verschiebt sich das Verhältnis auf etwa 4 : 1 zugunsten des Monoamids.

### Beispiele

### Beispiel 1

### Herstellung der offenkettigen Amide aus γ-BL und MMA oder DMA

Die Umsetzungen erfolgen in einem Rundkolben unter Eiskühlung, wobei die entsprechenden wässrigen Aminlösungen vorgelegt, und das γ-BL bei 5 °C Innentemperatur zugetropft wurde. Die Ausbeuten wurden jeweils über GC-F1.% ermittelt.

### 1a) Umsetzung von γ-BL mit MMA

- Ansatz:: 65 ml (0,75 mol) 40 %-ige MMA-Lösung
22 g (0,25 mol) γ-BL
- Ausbeute:: > 99,5 % N-Methyl-γ-hydroxybuttersäureamid

### 1b) Umsetzung von γ-BL mit DMA

- Ansatz:: 95 ml (0,75 mol) 40 %-ige DMA-Lösung
22 g (0,25 mol) γ-BL
- Ausbeute:: > 99,5 % N,N-Dimethyl-γ-hydroxybuttersäureamid

### 1c) Umsetzung von γ-BL mit einem MMA/DMA-Gemisch

- Ansatz:: 65 ml (0,75 mol) 40 %-ige MMA-Lösung
95 ml (0,75 mol) 40 %-ige DMA-Lösung
44 g (0,5 mol) γ-BL
- Ausbeuten:: 76,3 % N-Methyl-γ-hydroxybuttersäureamid
23,6 % N,N-Dimethyl-γ-hydroxybuttersäureamid

### Beispiel 2

Umsetzung von γ-BL mit MMA, DMA oder TMA zu NMP

### 2a) Einsatz der entsprechenden wässrigen Aminlösungen

Die Umsetzungen wurden in einem 300 ml-Autoklaven durchgeführt. Dabei wurde zunächst das entsprechende Amin als wässrige Lösung vorgelegt und γ-BL langsam zugegeben, wobei sich in einer exothermen Reaktion jeweils das entsprechende Amid (wie oben beschrieben), bzw. bei TMA ein entsprechendes inneres Salz (TMA-BL-Addukt) bildete. Nach Verschließen des Autoklaven wurden 20 bar N₂-Druck aufgepresst, auf 255 °C erhitzt und nach 3 Stunden wieder abkühlen gelassen. Die γ-BL-Umsätze und die entsprechenden NMP-Ausbeuten wurden jeweils über GC-F1.% ermittelt. Ergebnisse:

| Versuch | Amin | nicht umgesetztes γ-BL (%) | NMP-Ausbeute (%) |
|---|---|---|---|
| 1 | MMA | 0,05 | 98,2 |
| 2 | DMA | 13,2 | 63,1 |
| 3 | TMA | 48,3 | 22,1 |

### 2b) Einsatz der entsprechenden wasserfreien Amine

Die Umsetzungen wurden wie unter 2a) beschrieben in einem 300 ml-Autoklaven durchgeführt, wobei die Amine jeweils in den geschlossenen Autoklaven unter Druck einkondensiert wurden. Nach Zugabe von γ-BL wurde wie unter 2a) verfahren. Ergebnisse:

| Versuch | Amin | nicht umgesetztes γ-BL (%) | NMP-Ausbeute (%) |
|---|---|---|---|
| 1 | MMA | 0 | 95 |
| 2 | DMA | 31,8 | 21,4 |
| 3 | TMA | 95,0 | 0,1 |

### Beispiel 3

Umalkylierung von N,N-Dimethyl-γ-hydroxybuttersäureamid mit MMA.

In einem 500 ml Rundkolben wurden 65 g (0,5 mol) des Dimethylamids und 86 ml 40 %ige wässrige MMA-Lösung (1 mol) vorgelegt und eine Stunde lang auf 80 °C erwärmt. Im Austrag wurden 7,6 % des entsprechenden Monomethylamids detektiert.

### Beispiel 4

### Umsetzung von γ-BL mit einem MMA/DMA-Gemisch

In einem Rundkolben wurden je 0,75 mol MMA- und DMA-Lösung (40 %ig in Wasser) vorgelegt und bei 5 °C unter Eiskühlung mit γ-BL versetzt. Die Lösung wurde anschließend mit einer Verweilzeit von 10 Min. durch ein 270 °C heißes Rohr geleitet. Die Analyse der Austräge erfolgt jeweils über GC-Fl.%. Ergebnisse:

| Temperatur | entsprechendes Monomethylamid (Ausbeute in %) | entsprechendes Dimethylamid (Ausbeute in %) | NMP (Ausbeute in %) |
|---|---|---|---|
| 5°C | 76,3 | 22,6 | 0 |
| 270°C (10 Min.) | 40,8 | 10,2 | 45 |

### Beispiel 5

Umsetzung von γ-BL mit einem Methylamin-Gemisch.

Analog Beispiel 2 wurde ein Gemisch aus 0,1 mol MMA, 0,173 mol DMA und 0,29 mol TMA, jeweils als 40 %ige wässrige Lösung, mit 0,09 mol γ-BL 3 Stunden lang auf 255 °C erhitzt. Die Ausbeute an NMP betrug 94,8 % (0,0853 mol).

## Patentansprüche

1. Verfahren zur Herstellung von N-Methyl-2-pyrrolidon (NMP), **dadurch gekennzeichnet, dass** man in einer ersten Verfahrensstufe durch Reaktion von Ammoniak mit Methanol bei erhöhter Temperatur in Gegenwart eines Katalysators ein Gemisch enthaltend Mono-, Di- und Trimethylamin und Ammoniak herstellt, den Ammoniak abtrennt, das Gemisch enthaltend die Methylamine in einer zweiten Verfahrensstufe bei erhöhter Temperatur und erhöhtem Druck mit gamma-Butyrolacton (γ-BL), wobei das molare Verhältnis von Monomethylamin zu γ-BL mindestens 1 ist, umsetzt, NMP und unumgesetzte Methylamine aus dem Umsetzungsprodukt abtrennt und unumgesetztes Dimethylamin und Trimethylamin in die erste Verfahrensstufe zur Reaktion mit Methanol und Ammoniak zurückführt.

2. Verfahren nach Anspruch 1, **dadurch gekennzeichnet, dass** man die Reaktion der ersten Verfahrensstufe kontinuierlich bei einer Temperatur von 300 bis 500 °C und in Gegenwart eines festen sauren Katalysators durchführt.

3. Verfahren nach den Ansprüchen 1 oder 2, **dadurch gekennzeichnet, dass** das in der ersten Verfahrensstufe nach der Abtrennung von Ammoniak erhaltene Gemisch einen Gehalt an den drei Methylaminen, Wasser und Methanol von mindestens 96 Gew.-% aufweist.

4. Verfahren nach einem der Ansprüche 1 bis 3, **dadurch gekennzeichnet, dass** die Umsetzung der zweiten Verfahrensstufe bei einer Temperatur von 200 bis 300 °C durchgeführt wird.

5. Verfahren nach einem der Ansprüche 1 bis 4, **dadurch gekennzeichnet, dass** die Umsetzung der zweiten Verfahrensstufe bei einem Druck von 50 bis 150 bar durchgeführt wird.

6. Verfahren nach einem der Ansprüche 1 bis 5, **dadurch gekennzeichnet, dass** in der zweiten Verfahrensstufe das molare Verhältnis von Monomethylamin zu γ-BL 1,05 bis 1,5 beträgt.

7. Verfahren nach einem der Ansprüche 1 bis 6, **dadurch gekennzeichnet, dass** die Umsetzung der zweiten Verfahrensstufe kontinuierlich in einem Rohrreaktor mit einer Verweilzeit im Reaktor von 1,5 bis 5 Stunden durchgeführt wird.

8. Verfahren nach einem der Ansprüche 1 bis 7, **dadurch gekennzeichnet, daß** man in der ersten Verfahrensstufe ein Gemisch enthaltend Mono-, Di- und Trimethylamin im Gewichtsverhältnis MMA : DMA : TMA = (1 bis 14) : (6 bis 12) : (0,2 bis 12) herstellt.

## Claims

1. A process for preparing N-methyl-2-pyrrolidone (NMP), which comprises preparing a mixture comprising monomethylamine, dimethylamine and trimethylamine and ammonia in a first process step by reacting ammonia with methanol at elevated temperature in the presence of a catalyst, separating off the ammonia, reacting the mixture comprising the methylamines with gamma-butyrolactone (γ-BL), in a molar ratio of monomethylamine to γ-BL of at least 1 in a second process step at elevated temperature and superatmospheric pressure, separating NMP and unreacted methylamines from the reaction product and returning unreacted dimethylamine and trimethylamine to the first process step for reaction with methanol and ammonia.

2. A process as claimed in claim 1, wherein the reaction of the first process step is carried out continuously at from 300 to 500°C and in the presence of a solid acid catalyst.

3. A process as claimed in claim 1 or 2, wherein the mixture obtained in the first process step after separating off ammonia has a content of the three methylamines, water and methanol of at least 96% by weight.

4. A process as claimed in any of claims 1 to 3, wherein the reaction of the second process step is carried out at from 200 to 300°C.

5. A process as claimed in any of claims 1 to 4, wherein the reaction of the second process step is carried out at a pressure of from 50 to 150 bar.

6. A process as claimed in any of claims 1 to 5, wherein the molar ratio of monomethylamine to γ-BL in the second process step is from 1.05 to 1.5.

7. A process as claimed in any of claims 1 to 6, wherein the reaction of the second process step is carried out continuously in a tube reactor at a residence time in the reactor of from 1.5 to 5 hours.

8. A process as claimed in any of claims 1 to 7, wherein a mixture comprising mono-, di- and triethylamine in a weight ratio of MMA:DMA:TMA = (1 to 14):(6 to 12):(0.2 to 12) is prepared in the first process step.

## Revendications

1. Procédé de préparation de N-méthyl-2-pyrrolidone (NMP), **caractérisé en que**, dans une première étape du procédé, on prépare par réaction d'ammoniac avec du méthanol à température élevée, en présence d'un catalyseur, un mélange contenant de la monométhylamine, de la diméthylamine et de la triméthylamine et de l'ammoniac, on sépare l'ammoniac, on fait réagir le mélange contenant les méthylamines dans une deuxième étape du procédé, à température élevée et pression élevée, avec de la gamma-butyrolactone (γ-BL), le rapport molaire entre monométhylamine et γ-BL étant au moins de 1, on isole de la NMP et des méthylamines n'ayant pas réagi du produit de la réaction et on recycle la diméthylamine et la triméthylamine qui n'ont pas réagi dans la première étape du procédé pour réagir avec du méthanol et de l'ammoniac.

2. Procédé suivant la revendication 1, **caractérisé en ce qu'**on effectue la réaction de la première étape du procédé de manière continue à une température de 300 à 500°C et en présence d'un catalyseur acide solide.

3. Procédé suivant l'une des revendications 1 et 2, **caractérisé en ce que** le mélange obtenu dans la première étape du procédé, après la séparation de l'ammoniac, présente une teneur en les trois méthylamines, en eau et en méthanol d'au moins 96% en poids.

4. Procédé suivant l'une des revendications 1 à 3, **caractérisé en ce que** la réaction de la deuxième étape du procédé est effectuée à une température de 200 à 300°C.

5. Procédé suivant l'une des revendications 1 à 4, **caractérisé en ce que** la réaction de la deuxième étape du procédé est effectuée à une pression de 50 à 150 bars.

6. Procédé suivant l'une des revendications 1 à 5, **caractérisé en ce que**, dans la deuxième étape du procédé, le rapport molaire entre monométhylamine et γ-BL est de 1,05 à 1,5.

7. Procédé suivant l'une des revendications 1 à 6, **caractérisé en ce que** la réaction de la deuxième étape du procédé est effectuée de manière continue dans un réacteur tubulaire avec une durée de séjour dans le réacteur de 1,5 à 5 heures.

8. Procédé suivant l'une des revendications 1 à 7, **caractérisé en ce que**, dans la première étape du procédé, on prépare un mélange contenant de la monométhylamine, de la diméthylamine et de la triméthylamine dans un rapport pondéral MMA/DMA/TMA = (1 à 14)/(6 à 12)/(0,2 à 12).
